# EUROPEAN PATENT APPLICATION

(11) **EP 3 235 515 A1**
(43) Date of publication of application: **25.10.2017**
(21) Application number: 16166310.9
(22) Date of filing: 21.04.2016
(51) Int. Cl.: A61K 48/00, C12N 15/00, C12N 15/115

(54) **TARGETED MRNA FOR IN VIVO APPLICATION**

(71) Applicant: Eberhard Karls Universität Tübingen, 72074 Tübingen (DE)
(72) Inventor: Kormann, Michael, 72074 Tübingen (DE); Seitz, Christian, 72074 Tübingen (DE); Schlegel, Patrick, 72108 Rottenburg (DE)
(74) Representative: Witte, Weller & Partner Patentanwälte mbB

(57) **Abstract**

The present invention relates to a product for an *in vivo* expression of a protein in a living being, a medicament comprising said product, and to a nucleotide-modified mRNA for an *in vivo* expression of a protein in a living being.

SEQUENCE LISTING

<110> Eberhard-Karls-Universität Tübingen Medizinische Fakultät

<120> Targeted mRNA for in vivo application

<130> 5402P561EP

<160> 2

<170> BiSSAP 1.3

<210> 1
<211> 86
<212> RNA
<213> Artificial Sequence

<220>
<223> Anti-CD4 aptamer

<400> 1
gggagacaag aataaacgct caatgacgtc cttagaattg cgcattcctc acacaggatc     60

ttttcgacag gaggctcaca acaggc                                          86


<210> 2
<211> 2170
<212> RNA
<213> Artificial Sequence

<220>
<223> CD19-CD28-CD3-zeta construct

<400> 2
atgcttctcc tggtgacaag ccttctgctc tgtgagttac cacacccagc attcctcctg      60

atcccagaca tccagatgac acagactaca tcctccctgt ctgcctctct gggagacaga     120

gtcaccatca gttgcagggc aagtcaggac attagtaaat atttaaattg gtatcagcag     180

aaaccagatg gaactgttaa actcctgatc taccatacat caagattaca ctcaggagtc     240

ccatcaaggt tcagtggcag tgggtctgga acagattatt ctctcaccat tagcaacctg     300

gagcaagaag atattgccac ttacttttgc caacagggta atacgcttcc gtacacgttc     360

ggagggggga ctaagttgga aataacaggc tccacctctg gatccggcaa gcccggatct     420

ggcgagggat ccaccaaggg cgaggtgaaa ctgcaggagt caggacctgg cctggtggcg     480

ccctcacaga gcctgtccgt cacatgcact gtctcagggg tctcattacc cgactatggt     540

gtaagctgga ttcgccagcc tccacgaaag ggtctggagt ggctgggagt aatatggggt     600

agtgaaacca catactataa ttcagctctc aaatccagac tgaccatcat caaggacaac     660

tccaagagcc aagttttctt aaaaatgaac agtctgcaaa ctgatgacac agccatttac     720

tactgtgcca aacattatta ctacggtggt agctatgcta tggactactg gggtcaagga     780

acctcagtca ccgtctcctc agtagcagat cccgccgagc ccaaatctcc tgacaaaact     840

cacacatgcc caccgtgccc agcacctgaa ctcctggggg gaccgtcagt cttcctcttc 900

cccccaaaac ccaaggacac cctcatgatc tcccggaccc ctgaggtcac atgcgtggtg 960

gtggacgtga gccacgaaga ccctgaggtc aagttcaact ggtacgtgga cggcgtggag 1020

gtgcataatg ccaagacaaa gccgcgggag gagcagtaca acagcacgta ccgggtggtc 1080

agcgtcctca ccgtcctgca ccaggactgg ctgaatggca aggagtacaa gtgcaaggtc 1140

tccaacaaag ccctcccagc ccccatcgag aaaaccatct ccaaagccaa agggcagccc 1200

cgagaaccac aggtgtacac cctgccccca tcccgggatg agctgaccaa gaaccaggtc 1260

agcctgacct gcctggtcaa aggcttctat cccagcgaca tcgccgtgga gtgggagagc 1320

aatgggcagc cggagaacaa ctacaagacc acgcctcccg tgctggactc cgacggctcc 1380

ttcttcctct acagcaagct caccgtggac aagagcaggt ggcagcaggg gaacgtcttc 1440

tcatgctccg tgatgcatga ggctctgcac aaccactaca cgcagaagag cctctccctg 1500

tctccgggta aaaaagatcc caaattttgg gtgctggtgg tggttggtgg agtcctggct 1560

tgctatagct tgctagtaac agtggccttt attattttct gggtgaggag taagaggagc 1620

aggctcctgc acagtgacta catgaacatg actccccgcc gccccgggcc cacccgcaag 1680

cattaccagc cctatgcccc cccacgcgac ttcgcagcct atcgctccct gagagtgaag 1740

ttcagcagga gcgcagacgc ccccgcgtac cagcagggcc agaaccagct ctataacgag 1800

ctcaatctag gacgaagaga ggagtacgat gttttggaca agagacgtgg ccgggaccct 1860

gagatggggg gaaagccgag aaggaagaac cctcaggaag gcctgtacaa tgaactgcag 1920

aaagataaga tggcggaggc ctacagtgag attgggatga aaggcgagcg ccggaggggc 1980

aaggggcacg atggccttta ccagggtctc agtacagcca ccaaggacac ctacgacgcc 2040

cttcacatgc aggccctgcc ccctcgctaa tcctcgaggg gagacaagaa taaacgctca 2100

atgacgtcct tagaattgcg cattcctcac acaggatctt ttcgacagga ggctcacaac 2160

aggctccgga 2170

## Description

The present invention relates to a product for an *in vivo* expression of a protein in a living being, a medicament comprising said product, and to a nucleotide-modified mRNA for an *in vivo* expression of a protein in a living being.

Numerous genetic diseases are driven by the presence of dysfunctional or insufficiently expressed proteins. To this end, therapeutic approaches have aimed at delivering functionally active protein or its genetically encoded precursors, the corresponding DNA or messenger RNA (mRNA), into patients' cells. In theory, these methods allow for the expression of functionally active protein, leading to the correction of disease. Unfortunately, in practice, many obstacles remain to achieve a safe, long-term protein expression in a clinical setting.

Of these approaches, protein delivery is often hampered by protein metabolism or difficulty incorporating natural protein modifications, while gene delivery using plasmid DNA (pDNA) is commonly limited by CpG motifs that induce strong immune responses through innate immune receptors such as Toll-like receptor 9 (TLR9) and poor transfection efficiency in non- or slowly-dividing mammalian cells. Additionally, the use of viral vectors for gene therapy approaches has been threatened by the risk of insertional mutagenesis following random integration events that may occur within an oncogene or tumor suppressor. The development of immune responses against the viral capsid may also occur, which can prevent the possibility of vector re-administration.

For this reason, researchers have begun to explore the potential for mRNA to serve as an efficient, safe, non-integrating delivery vehicle for *in vivo* gene therapy. By micking endogenous mRNA modifications, the immune-stimulatory effects of *in vitro* transcribed mRNA can be prevented thus paving the way for transcript supplementation therapy as an efficient alternative to viral vectors. In 2011, it was demonstrated that *in vivo* delivery of nucleotide modified mRNA could achieve therapeutic levels of protein expression in murine lung; see Kormann, M.S., et al., Expression of therapeutic proteins after delivery of chemically modified mRNA in mice. Nat Biotechnol, 2011. 29(2): p. 154-7 and Mclvor, R.S., Therapeutic delivery of mRNA: the medium is the message. Molecular therapy : The Journal of the American Society of Gene Therapy, 2011, 19(5): p. 822-3. Unlike gene therapy with most viral vectors, the use of modified mRNA allowed for multiple administrations of therapeutic transcript, while providing a safer alternative that prevented immune activation and eliminated the possibility of genomic integration.

Uchida et al., Systemic delivery of messenger RNA for the treatment of pancreatic cancer using polyplex nanomicelles with a cholesterol moiety. Biomaterials 2016, 82: p. 221-8, describe the *in vivo* administration of mRNA encoding an anti-angiogenic protein (sFlt-1) packed in nanomicelles into mice suffering from a pancreatic tumor. The authors describe an inhibitory effect on the tumor growth.

However, so far the *in vivo* administration or delivery of mRNA is characterized by a low target-finding capacity. The administered mRNA is, in principle, spread all over the body or accumulated in a large number of tissues or the cardiovascular system, and only a low amount reaches the target cells. Therefore, the occurrence of side effects and systemic toxicity poses a severe problem in this approach.

A concept where an unspecific distribution of therapeutic or recombinant nucleic acid in a patient's body can be controlled to a better degree is the so-called adoptive cell transfer (ACT). ACT is the transfer of previously autologous or non-autologous withdrawn cells, most commonly derived from the immune system, into a patient, with the goal of transferring improved immune functionality and characteristics along with the cells back to the patient. The cells can be treated *ex vivo* with the recombinant nucleic acid or a vector encoding for a desired functionality by means of genetic engineering, or an immunogen or drug, thereby effecting an alteration or reprogramming of the withdrawn cells, before re-administering them into the patient. As of 2015 ACT had expanded to treat cervical cancer, lymphoma, leukemia, bile duct cancer and neuroblas-toma; see Rosenberg et al., Adoptive cell transfer as personalize immunotherapy for human cancer. Science 348 (6230): p. 62-68*,* and in 2016, lung cancer, breast cancer, sarcoma and melanoma. In 2016 CD19-specific chimeric antigen receptor (CAR)-modified T cells were used to treat patients with relapsed and refractory CD19+ B cell malignancies, including B cell acute lymphoblastic leukemia (B-ALL) harboring rearrangement of the mixed lineage leukemia (MLL) gene with CD19 CAR-T cells, see Gardner et al., Acquisition of a CD19 negative myeloid phenotype allows immune escape of MLL-rearranged B-ALL from CD19 CAR-T cell therapy. Blood: blood-2015-08-665547.

However, the ACT technology is very complex and expensive. The withdrawn cells are to be individualized and prepared for each and every patient separately. Therefore the ACT technology can only be provided by highly specialized clinics resulting in the exclusion of a large number of potentially needy patients.

Against this background the object underlying the invention is to provide a new product allowing the targeted expression of a protein in a living being, by means of which the disadvantages of the approaches and technologies in the prior art can be avoided or reduced, respectively.

This problem is solve by the provision of a product for an *in vivo* expression of a protein in a living being, comprising:
- a first entity comprising a first nucleic acid encoding an intracellularly expressible protein, and, associated therewith,
- a second entity configured for a specific binding to a cellular structure of said living being.

The inventors have surprisingly realized that with this product that combines the first entity which ensures the *in vivo* expression of any desired protein in a living being, and the second entity providing for the target-finding capacity, all features are embodied which are essential to effect a therapeutically useful phenomena in the living being.

According to the invention "product" refers to a biologically active molecule such as a nucleic acid, a peptide, a protein, a chemical structure, a macromolecule or a mixture thereof.

According to the invention a "protein" refers to a biomolecule or macromolecule consisting of one or more long chains of amino acid residues and explicitly includes a peptide and polypeptide.

The "first entity" and the "second entity" are functionally distinct units, not necessarily but possibly also structurally distinct units of the product according to the invention. Both entities are associated with each other, either by non-covalent or covalent binding, e.g. phosphodiester bonds in cases where not only the first entity comprises a (first) nucleic acid but also the second entity comprises a (second) nucleic acid molecule(s), e.g. in cases where both entities are realized by mRNA molecules. An example for a non-covalent association or binding is the nucleic acid hybridization again in cases where not only the first entity comprises a (first) nucleic acid but also the second entity comprises a (second) nucleic acid molecule(s).

The first and second entities may each be embodied by a single molecule, e.g. a single nucleic acid molecule. However, each of the entities may also be represented by two, three, four, five or more molecules.

While the "first entity" comprises a conventional nucleotide coding sequence the "second entity" may be a capture or binding molecule being specific and selective for the cellular structure and may be embodied, e.g., by an aptamer, an immunoglobulin, a mono- or polyclonal antibody etc. In case of the second entity being an aptamer the latter may comprise a second nucleotide sequence coding for a spatial motive allowing the specific binding to the cellular structure of said living being.

An "intracellularly expressible protein" as encoded by the first entity or the first nucleic acid, respectively, is a protein that, when the first entity or first nucleic acid is internalized into a cell of the living being, it is converted into a protein, e.g. by using the cellular translation machinery.

A "cellular structure" of said living being is a biological formation present in the living being, which the second entity can specifically bind to, such as an antigen or a surface receptor of a cell, e.g. an immune cell, including a T cell.

A "specific binding" refers to a selective binding of the second entity to the cellular structure as opposed to a non-specific, non-selective binding. An example for a specific binding is the binding of an aptamer or an antibody to their specific targets.

According to the invention a "living being" includes animals of all kinds and a human being.

The product according to the invention is configured for an expression *"in vivo*", i.e. for an administration or delivery into the living being and the expression in the living being's cell inside of the organism.

In contrast to the therapeutic approaches used so far in the state of the art based on *ex vivo* modifications of cells of the living being, such as the ATC technology, the product according to the invention can be produced "off the shelf" and by means of simple injection can be transferred into the patient. Moreover, the invention uses the living being's entire cell reservoir as the potential expression machinery but not only a few number of isolated and *ex vivo* prepared cells which then need to be re-infused. This results in a significantly improved expression or presence of the transferred protein coding sequence or transgen, respectively.

The second entity of the product according to the invention allows the targeted addressing of any cellular structures, such as specific cell sub-populations, like immune cells including T cells, e.g. T cells with a stem-cell like phenotype.

Another advantage of the product according to the invention is the transient expression of the protein encoded by the first nucleic acid. This allows the expression and, in turn, the biological activity of said protein being limited in time or controlled in time by an repetitive administration of the product according to the invention. Side effects are herewith reduced resulting in an improved safety profile.

The problem underlying the invention is herewith completely solved.

In a preferred embodiment of the invention said first nucleic acid is a messenger ribonucleic acid (mRNA), preferably a nucleotide-modified mRNA.

This measure has the advantage that the first entity is provided in a form allowing the direct transfer or translation of the encoded information into the desired protein by using the cellular protein synthesis machinery.

According to the invention "nucleotide-modified messenger RNA" refers to such an mRNA, where a part of the nucleotides, or nucleosides or nucleobases is modified or changed, respectively. In this respect the terms "nucleotides" and "nucleosides" are used interchangeably. Preferably it is referred to a chemical modification. This modification has the result that the mRNA is more stable and has less immunogenicity. Nucleotide-modified messenger RNA is generally known in the prior art, cf. for example WO 2011/012316. The content of the before-mentioned publication is incorporated herein by reference. Examples for chemically-modified nucleotides or nucleosides are pseudour-idine (Ψ), 5-methylcytidine (m5C), N6-methyladenosine (m6A), 5-methyluridine (m5U) or 2-thiouridine (s2U).

According to a preferred embodiment up to including approx. 100 % of the uridine nucleotides and/or up to including approx. 100 % of the cytidine nucleotides, preferably up to including approx. 70 % of the uridine nucleotides and/or up to including approx. 70 % of the cytidine nucleotides, further preferably up to including approx. 50 % of the uridine nucleotides and/or up to including approx. 50 % of the cytidine nucleotides, further preferably up to including approx. 25 % of the uridine nucleotides and/or up to including approx. 25 % of the cytidine nucleotides, and further preferably approx. 10 % of the uridine nucleotides and/or approx. 10 % of the cytidine nucleotides of the mRNA are modified, further preferably by exchanging uridine for 2-thiouridine (s2U) and/or pseudour-idine (Ψ) and/or by exchanging cytidine for 5-methylcytidine (m5C).

Further examples of chemically-modified nucleotides or nucleosides include thiouridine, N1-methylpseudouridine, 5-hydroxymethylcytidine, 5-hydroxymethylcytidine, 5-hydroxymethyluridine, 5-methylcytidine, 5-methoxyuridine, 5-methoxycytidine, 5-carboxymethylesteruridine, 5-formylcytidine, 5-carboxycytidine, 5-hydroxycytidine, thienoguanosine, 5-formyluridine. Each of theses chemically-modified nucleotides or nucleosides are, independently from and/or in combination with each other, suitable to replace its non-modified counterpart by up to approx. 10 %, preferably up to approx. 25 %, further preferably up to approx. 50 %, further preferably up to approx. 70 %, further preferably up to approx. 100 %. Preferred combinations are 5-methylcytidine/thiouridine; 5-hydroxymethylcytidine/5-hydroxymethyluridine; 5-methylcytidine/pseudouridine; 5-methoxyuridine/5-methyluridine; 5-hydroxymethylcytidine/N1-methylpseudouridine; 5-methylcytidine/N1-methylpseudouridine; 5-methylcytidine/5-carboxymethylesteruridine; 5-methoxycytidine/N1-methylpseudouridine; 5-hydroxymethylcytidine/5-methoxyuridine; 5-methylcytidine/thienoguanosine; 5-methylcytidine/5-formyluridine.

This measure has the advantage that through the prescribed content of nucleotide modifications an mRNA is provided which is significantly stable and little immunogenic. Even more, the inventors could surprisingly realize that it is sufficient if only up to including about 10 % to approx. 25 % of the non-modified nucleotides or nucleosides are replaced by their modified counterparts. The inventors could provide evidence that also such slightly modified mRNA is stable and little immunogenic. Since the nucleotide modification is complex, this has the advantage that the product according to the invention, because of the low concentration of nucleotide modifications, can be produced in a cost-saving manner. Besides of reducing costs, the lowering of the portion of modified nucleotides has also the advantage that the efficiency of the translation is increased. This is because very high portions of specifically modified nucleotides significantly interfere with the translation of the modified mRNA. However, with lower portions an optimum translation can be observed.

In another embodiment of the invention said intracellularly expressible protein is a protein capable of effecting at least one of the following phenomena in said living being in a targeted manner: immune response, cytokine expression, cell death induction, cell death inhibition, transcription factor expression, genetic modification, epigenetic modification.

The person of skill in the art is perfectly aware of the type of expressible protein to be chosen to achieve the intended effect.

"Cell death" is to be understood as encompassing all phenomena resulting in the dying of cells, including apoptosis.

This measure has the advantage that the product according to the invention initiates biological responses in the living being which trigger a therapeutically useful effect.

In another embodiment of the product according to the invention said intracellularly expressible protein is an antigen-specific receptor.

This measure has the advantage that an antigen specific receptor, in particular if expressed in an immune cell, allows the targeted induction of an immune response specifically directed against the respective antigen. In doing so, the immune cell of the living being is re-programmed to activate the adaptive immune system against the antigen.

In another embodiment of the product according to the invention said antigen-specific receptor is selected from the group consisting of:
- T-cell receptor, preferably a tumor antigen-specific T-cell receptor, a virus antigen specific T-cell receptor, a bacterium antigen-specific T-cell receptor, a fungus antigen-specific T-cell receptor; or
- a chimeric antigen receptor (CAR),
   - preferably a CAR targeting a tumor-associated antigen, preferably selected from the group consisting of: HER2/neu, ErbB, EGFR, EG-FRvlll, FGFR3, FGFR4, LI-13R, II-13Rα2, II-11Rα, VEGFR2, ALK, GD2, GD3, mesothelin, Survivin, PMSA, PSCA, CEA, MUC1, GPC3, GPC5, CSPG4, ROR1, FR-α, FR-β, Igk, Lewis^{Y}, Glypican3, EphA2, CAIX, CSPG4, AFP, FAP, c-MET, HLA-DR, CA-125, CS1, BCMA, NKG2D ligands (MICA/MICB), CD10, CD19, CD20, CD22, CD30, CD33, CD34, CD38, CD44, CD44v6, CD44v7/8, CD123, CD133, CD135, CD138, CD152, CD171; or
   - a CAR targeting a bacterium specific antigen, and/or
   - a CAR targeting a fungus specific antigen, and/or
   - a CAR targeting a virus specific antigen.

This measure has the advantage that such an antigen-specific receptor is encoded which has the capability to induce an effective and targeted immune response against the respective antigen.

In a further embodiment of the invention said intracellularly expressible protein is
- a cell death inducing or inhibiting protein, preferably selected from the group consisting of: a caspase, a second mitochondria-derived activator of caspases (SMAC), a BCL-2 family protein, an inhibitor of apoptosis protein (IAP), a tumor necrosis factor receptor superfamily (TNFRSF) protein, the death-inducing signaling complex, p53, and interferons, or
- an immune modulatory protein, preferably selected from the group consisting of: cytokines, chemokines, tumor necrosis factor (TNF) family proteins and colony stimulating factors, or
- a gene expression or protein modulating cellular signaling molecule, preferably selected from a group consisting of: kinases, phosphatases, acetyltransferases, deacetylases, methyltransferases, SUMOylating enzymes, and deSUMOylating enzymes, or
- a gene sequence modulating molecule, preferably selected from the group consisting of: zinc-finger nucleases, meganucleases, TAL effector nucleases, CRISPR/Cas9 related nucleases, nickases, and *Fok*I based dCas9 nucleases.

This measure allows the targeted treatment of diseases characterized by degenerated cells. Such degenerated cells will be subjected to cell death or apoptosis resulting in its specific killing, however without involving non-degenerated or healthy cells, respectively. Alternatively, cell death can be purposefully inhibited, the immune response or cellular signaling or gene sequence can be modulated in a targeted fashion.

Example of suitable cytokines include interleukins IL-1, -2, -3, -4, -5, -6, - 7, -8, -9, -10, -11, -12, -13, -14, -15, -16, -17, -18, -19, -20, -21, -22, -23, -24, -25, -26, -27, -28, -29, -30, -31, -32, -33, -34, -35, and IL-36. Chemokines include CCL1, -2, -3, -4, -5, - 6, -7, -8, -9, -10, -11, -12, -13, -14, -15, -16, -17, -18, -19, -20, -21, -22, -23, -24, -25, -26, - 27, and CCL28; CXCL1 to CXCL17; XCL1, XCL2; CX3CL1; RANTES; type I interferons; IFN-α, -β, -ε, -κ, -τ, -δ, -ζ, -ω, -ν, -v; type II interferon; IFN-γ; type III interferons. Tumor necrosis factor (TNF) family proteins include TNFa, TNFβ, CD40L, CD27L, CD30L, FASL, 4-1 BBL, Ox40L, TRAIL. Colony stimulating factors include M-CSF, G-CSF and GM-CSF. Other immune stimulatory or inhibitory proteins can also be chosen such as CD70, CD80, CD86, ICOSL, PD-L1, PD-L2.

In an embodiment the gene expression or protein modulating cellular signaling molecules also include general activators or inhibitors of gene transcription or translation or cell surface and/or intracellular signal transducing receptors.

In another embodiment of the product according to the invention said cellular structure which can be bound by the second entity is a cell surface molecule, preferably a cell surface expressed protein, further preferably a protein characterizing a cell of the human hematopoiesis and/or a cell of the human immune system, i.e. an immune cell.

With this measure the invention takes advantage of such cellular structures which function as biomarkers allowing the specific addressing of individual sub-populations of cells. Such subpopulations expressing the respective cell surface molecule will, when being bound by the second entity, e.g. the aptamer or antibody, internalize the product according to the invention or, at least, the first entity comprising the first nucleotide acid and ensure that the encoded protein will by expressed.

In another embodiment of the invention said cell surface expressed protein recognized and bound by the second entity is a cluster of differentiation (CD) protein or equivalent, preferably said CD protein is selected from the group consisting of: CD4, CD8, CD3, CD10, CD16, CD19, CD20, CD22, CD25, CD28, CD30, CD33, CD34, CD38, CD44, CD44v6, CD44v7/8, CD45, CD45RA, CD45RO, CD56, CD62L, CD95, CD123, CD127, CD133, CD135, CD137, CD138, CD152, CD171, and preferably said equivalent is selected from the group consisting of: CCR4, CCR5, CCR6, CCR7, CXCR3, CXCR4, CXCR5, TCRαβ, TCRγδ, CTLA-4, PD1, TIM3, NKG2D, HER2/neu, ErbB, EGFR, EG-FRvlll, FGFR3, FGFR4, LI-13R, 11-13Rα2, II-11 Rα, VEGFR2, ALK, GD2, GD3, mesothelin, survivin, PMSA, PSCA, CEA, MUC1, GPC3, GPC5, CSPG4, ROR1, FR-α, FR-β, Igk, Lewis^{Y}, glypican3, EphA2, CAIX, CSPG4, AFP, FAP, c-MET, HLA-DR, CA-125, CS1, BCMA, NKG2D ligands (MICA/MICB).

This measure makes use of such surface expressed proteins which characterize and identify immune cells, thereby allowing a specific delivery of the product to immune cells.

According to another embodiment of the product of the invention said second entity is an aptamer.

This measure has the advantage that such a binding molecule is used with can be generated against almost any target structure of interest. Further, it can be easily associated with the first entity, e.g. by attaching it to the first entity or first nucleic acid/first nucleotide sequence via the simple concatenation of nucleotides of the first nucleic acid and nucleotides of the aptamer, e.g. via a phosphodiester bonds. This can be easily realized by methods of nucleic acid synthesis. The aptamer can be attached to the 5' end or the 3' end of the first nucleic acid or, if more than one aptamer is used, e.g. two aptamers, one can be attached to the 5' end and another can be attached to the 3' end. Alternatively, the aptamer can be associated with the first nucleic acid by hybridizing nucleotides of the aptamer to complementary nucleotides of the first nucleic acid.

Therefore, it is a preferred embodiment where said aptamer is an RNA aptamer, i.e. an aptamer consisting of or comprising ribonucleotides.

This measure allows an easy synthesis of the product according to the invention in form of a single ribonucleic acid molecule comprising the first nucleotide sequence encoding the intracellularly expressible protein and a second nucleotide sequence encoding the aptamer.

As a consequence, in another embodiment of the invention said aptamer is connected to said first nucleic acid by the concatenation of nucleotides resulting in a single-stranded nucleic acid molecule, preferably a single-stranded mRNA molecule, or wherein said aptamer is connected to said nucleic acid by the hybridization of complementary bases resulting in a double-stranded nucleic acid molecule, preferably a double-stranded mRNA molecule.

In another embodiment the product according to the invention comprises nanoparticles complexed with said first and/or said second entity and/or comprises liposomes packaging said first and/or said second entity.

As the inventors were able to realize, the complexation of the first and/or second entity with a nanoparticle, e.g. a chitosan-coated PLGA nanoparticle, or the packaging into liposomes significantly increases the degree of internalization of the product according to the invention into the target cells.

Another subject-matter of the invention relates to the product specified above for the treatment a disease, preferably selected from the group consisting of: a tumor and/or oncologic disease, a hematologic disease, an infectious disease, a rheumatologic disease, a genetic/hereditary disease, an autoimmune disease, an allergic disease.

The features, characteristics and advantages of the product according to the invention apply likewise to this subject-matter.

Another subject-matter relates to a medicament comprising the product according to the invention and a pharmaceutically acceptable carrier.

For this purpose, a "pharmaceutically acceptable carrier" is understood to mean any excipient, additive, or vehicle that is typically used in the field of the treatment of the mentioned diseases and which simplifies or enables the administration of the product according to the invention to a living being, and/or improves its stability and/or activity. The pharmaceutical composition can also incorporate binding agents, diluting agents or lubricants. The selection of a pharmaceutical carrier or other additives can be made on the basis of the intended administration route and standard pharmaceutical practice. As pharmaceutical acceptable carrier use can be made of solvents, extenders, or other liquid binding media such as dispersing or suspending agents, surfactant, isotonic agents, spreaders or emulsifiers, preservatives, encapsulating agents, solid binding media, depending upon what is best suited for the respective dose regime and is likewise compatible with the compound according to the invention. An overview of such additional ingredients can be found in, for example, Rowe (Ed.) et al.: Handbook of Pharmaceutical Excipients, 7th edition, 2012, Pharmaceutical Press.

The features, characteristics and advantages of the product according to the invention apply likewise to the medicament according to the invention.

Another subject-matter of the invention relates to a nucleotide-modified mRNA for an *in vivo* expression of a protein in a living being comprising:
- a first ribonucleotide sequence encoding an intracellularly expressible protein, and
- a second ribonucleotide sequence encoding an aptamer configured for a specific binding to a cellular structure of said living being.

The features, characteristics and advantages of the product according to the invention apply likewise to the nucleotide-modified mRNA according to the invention and *vice versa.*

Another subject-matter relates to a method for the treatment of a disease, preferably a tumor and/or oncologic disease, a hematologic disease, an infectious disease, a rheumatologic disease, a genetic/hereditary disease, an autoimmune disease, an allergic disease, comprising the administration of the product according to the invention and/or the medicament according to the invention into a living being.

In a further embodiment of the method according to the invention the product and/or medicament is administered intravenously (i.v.). In embodiments it comprises a single and/or multiple administrations and continuous administration, e.g. via a drip or pump.

The features, characteristics and advantages of the product according to the invention apply likewise to the method according to the invention and *vice versa.*

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention.

The features mentioned in the specific embodiments are also features of the invention in general, which are not only applicable in the respective embodiment but also in an isolated manner in the context of any embodiment of the invention.

The invention is also described and explained in further detail by referring to the following drawings:
- Fig. 1: illustrates therapeutic approaches of the state of the art aimed at delivering functionally active protein or its precursors, exemplified by cystic fibrosis transmembrane conductance regulator (CFTR), into patient's cells;
- Fig. 2: shows the result of an analysis by means of cytometry of blood cells withdrawn from mice after the administration *in vivo* of nucleotide-modified mRNA encoding red fluorescent reporter protein (RFP) assembled to nanoparticles into mice for the expression of RFP;
- Fig. 3: illustrates the *in vivo* immune reaction represented by IFN-alpha as measured by ELISA, initiated in mice after the administration *in vivo* of nucleotide-modified mRNA encoding RFP assembled to nanoparticles;
- Fig. 4: shows the result of an analysis by means of cytometry of immune (CD4+) cells withdrawn from mice after the administration *in vivo* of aptamer-targeted nucleotide-modified mRNA (atmRNA) encoding RFP and an anti-CD4 aptamer assembled to nanoparticles into mice for the expression of RFP;
- Fig. 5: illustrates the structure of the an atmRNA consiting of an aptamer (ar) targeting an effector antigen (ea) and a transgen (tg) (e.g. a chimeric antigen receptor) that is encoded by modified mRNA (mr).
- Fig. 6: shows the result on an analysis by means of cytometry of immune (CD4+) cells withdrawn from mice after the administration *in vivo* of atmRNA encoding anti-CD19 CAR and an anti-CD4 aptamer assembled to nanoparticles into mice for the expression of anti-CD19 CAR.

### Embodiments

### 1. Methods of the prior art

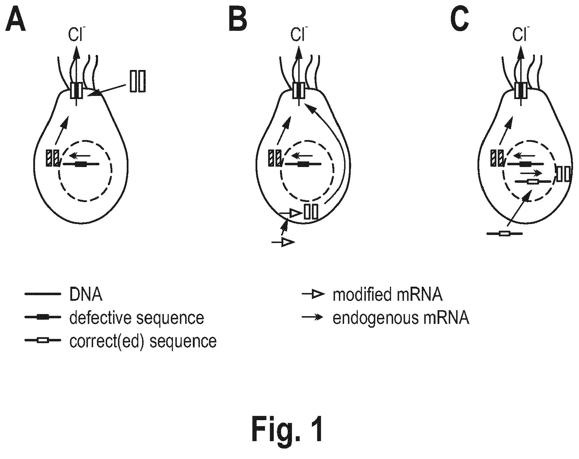

Fig. 1 illustrates methods of the art for restoring functional protein expression in the setting of genetic disease. In this example, a genetic mutation in the cystic fibrosis transmembrane conductance regulator gene, *CFTR,* leads to faulty expression of the CFTR protein, a chloride ion channel anchored in the plasma membrane. By supplementing the cell with functional CFTR protein (A), *CFTR* cDNA (B), or mRNA transcripts (C), there is potential to overcome the genetic defects underlying this disease.

In the protein supplementation therapy shown in (A) a correct version of CFTR is transfected or transduced into the respective target cells. The protein delivery is often ineffective and it is difficult to include all natural post-protein modifications. In the transcript supplementation therapy shown in (B) a correct version of *CFTR*-mRNA is transfected into the respective target cells. Note the mRNA is actively producing CFTR already in the cytoplasm, thereby circumventing the nuclear membrane. In the gene supplementation therapy shown in (C) a correct version of the *CFTR* gene is transfected or transduced into the respective target cells. Note that the DNA has to enter the nucleus to be transcribed, which is a major barrier in gene therapy. Furthermore, gene delivery using plasmid DNA is commonly limited by CpG motifs that induce strong immune responses through innate immune receptors such as Toll-like receptor 9 (TLR9) and poor transfection efficiency in non- or slowly-dividing mammalian cells. Additionally, the use of viral vectors for gene therapy approaches has been threatened by the risk of insertional mutagenesis following random integration events that may occur within an oncogene or tumor suppressor. The development of immune responses against the viral capsid may also occur, which can prevent the possibility of vector re-administration.

### 2. Design of an aptamer targeted mRNA (atmRNA)

The inventors have developed a nucleotide-modified mRNA that can be delivered intravenously (i.v.) in mice to reprogram T cells, thus targeting a specific antigen. The antigen may be located on tumor cells, viruses, bacteria or funghi.

The disclosed mRNA consists of a part that encodes for a chimeric antigen receptor (CAR) and it consists - downstream of the CAR encoding part - of a non-protein coding aptamer sequence. The aptamer sequence is able to target e.g. CD4+ T cells.

The DNA sequence encoding the anti-CD4 aptamer is as follows:

5'-GGGAGACAAGAATAAACGCTCAATGACGTCCTTAGAATTGCGCA
TTCCTCACACAGGATCTTTTCGACAGGAGGCTCACAACAGGC-3' (SEQ ID No. 1).

In the corresponding mRNA sequence of the anti-CD4 aptamer each "T" (thymine) is replaced by an "U" (uracil).

As the aptamer is critical for the specificity of the mRNA, the inventors call this mRNA "atmRNA" (aptamer targeted mRNA).

### 3. Functional tests demonstrating the activity of the atmRNA

First, the inventors tested such an mRNA using a red fluorescent reporter protein (RFP) as the encoding part without aptamer sequence. For this experiment RFP DNA was subcloned into the pVAX-A120 vector; see Kormann et al (I.c.). For *in vitro* transcription (IVT) of chemically modified mRNA the plasmid was linearized with *Xho*I and transcribed *in vitro* using the MEGAscript T7 Transcription kit (www.lifetechnologies.com), incorporating 25% 2-thio-UTP and 25% 5-methyl-CTP or 100% PseudoUTP and 100% 5-methyl-CTP (all from www.trilinkbiotech.com). The anti reverse CAP analog (ARCA)-capped synthesized nec-mRNAs were purified using the MEGAclear kit (www.lifetechnologies.com) and analyzed for size on agarose gels and for purity and concentration on a NanoPhotometer (http://www.implen.com),

In the first experiment (modified RFP-mRNA) the inventors assembled the mRNA to nanoparticles (NPs) with Chitosan-coated PLGA using the following protocol: Chitosan (83% deacetylated (Protasan UP CL 113, www.novamatrix.biz)) coated PLGA (poly-d,l-lactide-co-glycolide 75:25 (Resomer RG 752H, www.evonik.de) nanoparticles (short: NPs) were prepared by using emulsion-diffusion-evaporation15 with minor changes. In brief, 100 mg PLGA was dissolved in ethyl acetate and added dropwise to an aqueous 2.5% PVA solution (polyvinyl alcohol, Mowiol 4-88, www.kuraray.eu) containing 15 mg Chitosan. This emulsion was stirred (1.5 h at room temperature) and followed by homogenization at 17,000 r.p.m. for 10 min using a Polytron PT 2500E (www.kinematica.ch). These positively charged NPs were sterile filtered and characterized by Malvern ZetasizerNano ZSP (hydrodynamic diameter: 157.3 Å} 0.87 nm, PDI 0.11, zeta potential +30.8 Å} 0.115 mV). After particle formation they were loaded with mRNA by mixing (weight ratio, 25:1).

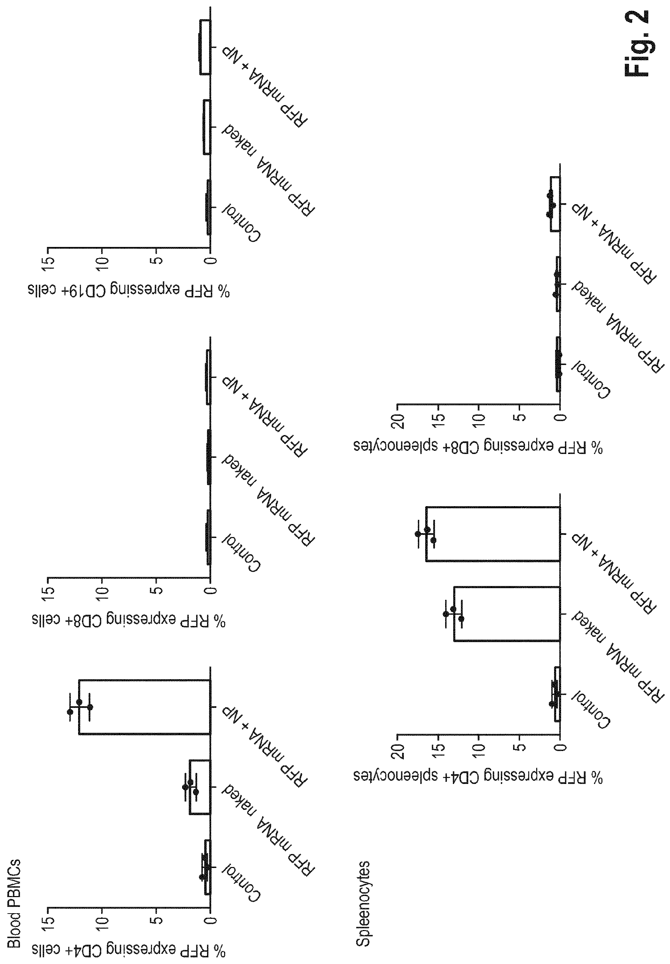

20 µg modified RFP-mRNA-NPs in a total volume of 100 µl were administered i.v. into the tail vein of BALB/c mice. After 24 h blood was withdrawn retroorbitally and immune cells were analyzed for RFP expression by flow cytometry. The data is shown in Figure 2: RFP+ cells were determined and quantified via flow cytometry. In some cell contexts RFP mRNA + NP was significantly higher expressed compared to RFP mRNA alone (n=3 mice per group). **P*<0,05, ****P*<0,001 (Mann-Whitney-U tests).

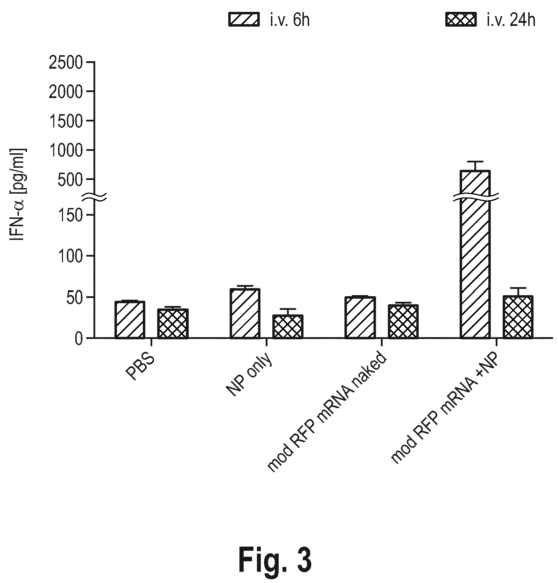

The immune reaction developed upon i.v. administration of RFP-mRNA-NPs was measured via quantification of IFN-alpha release after 6 h and 24 h using ELISA, as shown in Figure 3: In vivo immune reaction to chemically modified RFP mRNA complex to NPs. 20 µg of RFP mRNA with or without NPs was i.v. injected into mice (n=3 mice per group). 6 h and 24 h post-injection, IFN-alpha was measured by ELISA in duplicates.

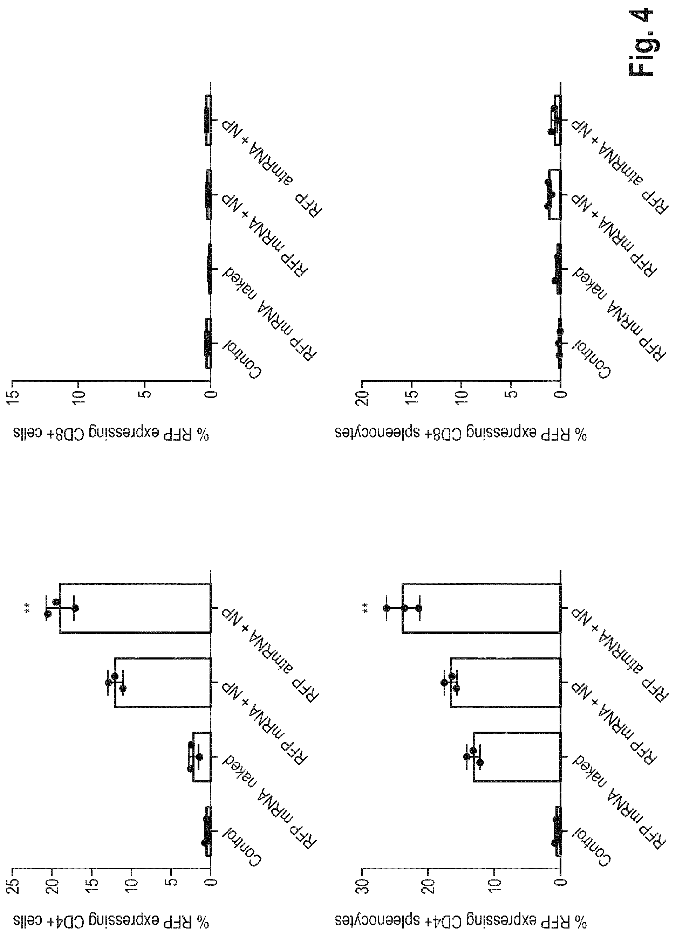

In a next experiment anti-CD4 aptamer sequence was added downstream of the polyA sequence. IVT was performed as described above to obtain atmRNA. i.v. application of the atmRNA to BALB/c mice was performed as described above. After 24 h blood was withdrawn retroorbitally and immune cells were analyzed for RFP expression bei flow cytometry. The data is shown in Figure 4: RFP+ cells were determined and quantified via flow cytometry. In some cell contexts RFP atmRNA + NP was significantly higher expressed compared to regular RFP mRNA + NP (n=3 mice per group). ***P*<0,01 (Mann-Whitney-U tests).

Clearly, CD4+ T cells showed a significantly higher expression of RFP compared to the expression found when using chemically modified RFP mRNA without attached aptamer sequence.

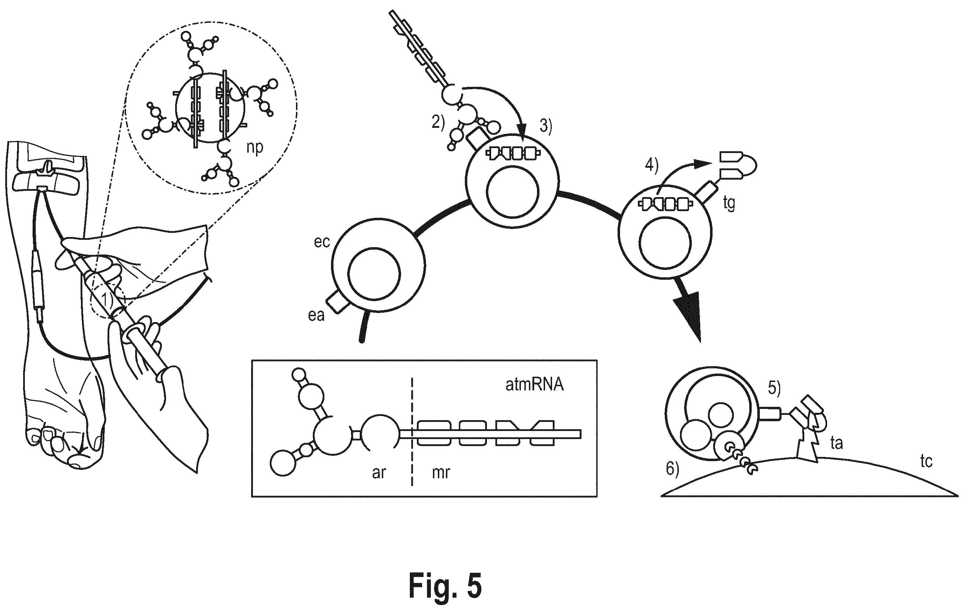

In a further experiment, RFP was substituted with a CD19-CD28-CD3-zeta construct, which - upon translation - assembles to an anti-CD19 CAR. The rationale behind that approach is depicted in Figure 5: The aptamer-targeted modified messenger-RNA (atmRNA) consists of an aptamer (ar) targeting an effector antigen (ea) and a transgen (tg) (e.g. a chimeric antigen receptor) that is encoded by modified mRNA (mr). 1) atmRNA complexed with a nanoparticle (np) is injected intravenously. 2) atmRNA binds to the effector antigen (ea) and to an effector cell (ec) (e.g. CD4 on a T cell). 3) atmRNA bound on an effector antigen (ea) is internalized due to antigen flux. 4) transgen (tg) encoding modified messenger RNA gets translated in the cytosol of the target cell (tc) and the transgen (tg) gets expressed on the target cell (tg). 5) The transgen (e.g. a chimeric antigen receptor) binds to the target antigen (e.g. tumor associated antigen) on a target cell (tc) (e.g. tumor cell). 6) The effector cell (e.g. chimeric antigen expressing T cell) gets activated and mediates functions to the target cell (tc) (e.g. induces cell death in a tumor cell).

The DNA sequence encoding the CD19-CD28-CD3-zeta construct is as follows:

5'-ATGCTTCTCCTGGTGACAAGCCTTCTGCTCTGTGAGTTACCACAC
CCAGCATTCCTCCTGATCCCAGACATCCAGATGACACAGACTACATCCTCCCTGTCT
GCCTCTCTGGGAGACAGAGTCACCATCAGTTGCAGGGCAAGTCAGGACATTAGTAAA
TATTTAAATTGGTATCAGCAGAAACCAGATGGAACTGTTAAACTCCTGATCTACCATA
CATCAAGATTACACTCAGGAGTCCCATCAAGGTTCAGTGGCAGTGGGTCTGGAACAG
ATTATTCTCTCACCATTAGCAACCTGGAGCAAGAAGATATTGCCACTTACTTTTGCCA
ACAGGGTAATACGCTTCCGTACACGTTCGGAGGGGGGACTAAGTTGGAAATAACAG
GCTCCACCTCTGGATCCGGCAAGCCCGGATCTGGCGAGGGATCCACCAAGGGCGA
GGTGAAACTGCAGGAGTCAGGACCTGGCCTGGTGGCGCCCTCACAGAGCCTGTCC
GTCACATGCACTGTCTCAGGGGTCTCATTACCCGACTATGGTGTAAGCTGGATTCGC
CAGCCTCCACGAAAGGGTCTGGAGTGGCTGGGAGTAATATGGGGTAGTGAAACCAC
ATACTATAATTCAGCTCTCAAATCCAGACTGACCATCATCAAGGACAACTCCAAGAGC
CAAGTTTTCTTAAAAATGAACAGTCTGCAAACTGATGACACAGCCATTTACTACTGTG
CCAAACATTATTACTACGGTGGTAGCTATGCTATGGACTACTGGGGTCAAGGAACCT
CAGTCACCGTCTCCTCAGTAGCAGATCCCGCCGAGCCCAAATCTCCTGACAAAACTC
ACACATGCCCACCGTGCCCAGCACCTGAACTCCTGGGGGGACCGTCAGTCTTCCTC
TTCCCCCCAAAACCCAAGGACACCCTCATGATCTCCCGGACCCCTGAGGTCACATGC
GTGGTGGTGGACGTGAGCCACGAAGACCCTGAGGTCAAGTTCAACTGGTACGTGGA
CGGCGTGGAGGTGCATAATGCCAAGACAAAGCCGCGGGAGGAGCAGTACAACAGC
ACGTACCGGGTGGTCAGCGTCCTCACCGTCCTGCACCAGGACTGGCTGAATGGCAA
GGAGTACAAGTGCAAGGTCTCCAACAAAGCCCTCCCAGCCCCCATCGAGAAAACCA
TCTCCAAAGCCAAAGGGCAGCCCCGAGAACCACAGGTGTACACCCTGCCCCCATCC
CGGGATGAGCTGACCAAGAACCAGGTCAGCCTGACCTGCCTGGTCAAAGGCTTCTA
TCCCAGCGACATCGCCGTGGAGTGGGAGAGCAATGGGCAGCCGGAGAACAACTAC
AAGACCACGCCTCCCGTGCTGGACTCCGACGGCTCCTTCTTCCTCTACAGCAAGCTC
ACCGTGGACAAGAGCAGGTGGCAGCAGGGGAACGTCTTCTCATGCTCCGTGATGCA
TGAGGCTCTGCACAACCACTACACGCAGAAGAGCCTCTCCCTGTCTCCGGGTAAAAA
AGATCCCAAATTTTGGGTGCTGGTGGTGGTTGGTGGAGTCCTGGCTTGCTATAGCTT
GCTAGTAACAGTGGCCTTTATTATTTTCTGGGTGAGGAGTAAGAGGAGCAGGCTCCT
GCACAGTGACTACATGAACATGACTCCCCGCCGCCCCGGGCCCACCCGCAAGCATT

ACCAGCCCTATGCCCCCCCACGCGACTTCGCAGCCTATCGCTCCCTGAGAGTGAAG
TTCAGCAGGAGCGCAGACGCCCCCGCGTACCAGCAGGGCCAGAACCAGCTCTATAA
CGAGCTCAATCTAGGACGAAGAGAGGAGTACGATGTTTTGGACAAGAGACGTGGCC
GGGACCCTGAGATGGGGGGAAAGCCGAGAAGGAAGAACCCTCAGGAAGGCCTGTA
CAATGAACTGCAGAAAGATAAGATGGCGGAGGCCTACAGTGAGATTGGGATGAAAG
GCGAGCGCCGGAGGGGCAAGGGGCACGATGGCCTTTACCAGGGTCTCAGTACAGC
CACCAAGGACACCTACGACGCCCTTCACATGCAGGCCCTGCCCCCTCGCTAATCCT
CGAGGGGAGACAAGAATAAACGCTCAATGACGTCCTTAGAATTGCGCATTCCTCACA
CAGGATCTTTTCGACAGGAGGCTCACAACAGGCTCCGGA-3' (SEQ ID No. 2).

In the corresponding mRNA sequence of the anti-CD4 aptamer each "T" (thymine) is replaced by an "U" (uracil).

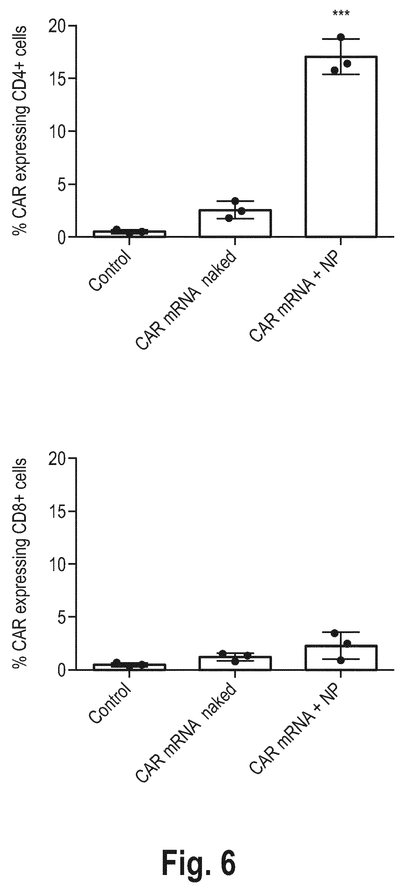

IVT of atmRNA encoding the anti-CD19 CAR was performed as described above. The atmRNA was i.v. injected into BALB/c mice. After 24 h blood was withdrawn retroorbitally and immune cells were analyzed for CAR expression bei flow cytometry. The data is shown in Figure 6: CAR+ cells were determined and quantified via flow cytometry. In CD4+ cells, CAR mRNA+ NP was significantly higher expressed compared to CAR mRNA alone (n=3 mice per group). ****P*<0,001, CAR mRNA + NP vs. CAR mRNA naked in CD4+ cells (Mann-Whitney-U tests).

The data conclusively demonstrates that CARs can be expressed on specific immune cells such as CD4+ T cells. Using nanoparticles or liposomes i.v. application is made possible, which overcomes all complicated and time-consuming *ex vivo* steps currently state-of-the-art to reprogram T cells. Thus, with the presented invention the genetic engineering of "designer T cells" can be done ultra-quick and "off-the-shelf" and opens up a vast range of possibilities to initiate / elicit antigen specific immune responses against cancer and infectious diseases.

### Sequences

SEQ ID no. 1: DNA sequence encoding the anti-CD4 aptamer;
SEQ ID no. 2: DNA sequence encoding the CD19-CD28-CD3-zeta construct

## Claims

1. Product for an *in vivo* expression of a protein in a living being, comprising
- a first entity comprising a first nucleic acid encoding an intracellularly expressible protein, and, associated therewith,
- a second entity configured for a specific binding to a cellular structure of said living being.

2. Product of claim 1, wherein said first nucleic acid is an mRNA, preferably a nucleotide-modified mRNA.

3. Product of any of the preceding claims, wherein said intracellularly expressible protein is a protein capable of effecting at least one of the following phenomena in said living being in a targeted manner: immune response, cytokine expression, cell death induction, cell death inhibition, transcription factor expression, genetic modification, epigenetic modification.

4. Product of claim 3, wherein said intracellularly expressible protein is an antigen-specific receptor, wherein preferably said antigen-specific receptor is selected from the group consisting of:
- T-cell receptor, preferably a tumor antigen-specific T-cell receptor, a virus antigen specific T-cell receptor, a bacterium antigen-specific T-cell receptor, a fungus antigen-specific T-cell receptor;
- a chimeric antigen receptor (CAR), further preferably a CAR targeting a tumor-associated antigen, further preferably selected from the group consisting of: HER2/neu, ErbB, EGFR, EGFRvIII, FGFR3, FGFR4, LI-13R, II-13Rα2, II-11Rα, VEGFR2, ALK, GD2, GD3, mesothelin, Survivin, PMSA, PSCA, CEA, MUC1, GPC3, GPC5, CSPG4, ROR1, FR-α, FR-β, Igk, Lewis^{Y}, Glypican3, EphA2, CAIX, CSPG4, AFP, FAP, c-MET, HLA-DR, CA-125, CS1, BCMA, NKG2D ligands (MICA/MICB), CD10, CD19, CD20, CD22, CD30, CD33, CD34, CD38, CD44, CD44v6, CD44v7/8, CD123, CD133, CD135, CD138, CD152, CD171; further preferably a CAR targeting a bacterium specific antigen, and/or further preferably a CAR targeting a fungus specific antigen, and/or further preferably a CAR targeting a virus specific antigen.

5. Product of claim 3, wherein said intracellularly expressible protein is
- a cell death inducing or inhibiting protein, preferably selected from the group consisting of: a caspase, a second mitochondria-derived activator of caspases (SMAC), a BCL-2 family protein, an inhibitor of apoptosis protein (IAP), a tumor necrosis factor receptor superfamily (TNFRSF) protein, the death-inducing signaling complex, p53, and interferons, or
- an immune modulatory protein, preferably selected from the group consisting of: cytokines, chemokines, tumor necrosis factor (TNF) family proteins and colony stimulating factors, or
- a gene expression or protein modulating cellular signaling molecule, preferably selected from a group consisting of: kinases, phosphatases, acetyltransferases, deacetylases, methyltransferases, SUMOylating enzymes, and de-SUMOylating enzymes, or
- a gene sequence modulating molecule, preferably selected from the group consisting of: zinc-finger nucleases, meganucleases, TAL effector nucleases, CRISPR/Cas9 related nucleases, nickases, and *Fok*I based dCas9 nucleases.

6. Product of any of the preceding claims, wherein said second entity is configured for a specific binding to a cell surface molecule, preferably a cell surface expressed protein, further preferably a protein characterizing a cell of the human hematopoiesis and/or a cell of the human immune system.

7. Product of claim 6, wherein said cell surface expressed protein is a cluster of differentiation (CD) protein or equivalent.

8. Product of claim 7, wherein said CD protein is selected from the group consisting of: CD4, CD8, CD3, CD10, CD16, CD19, CD20, CD22, CD25, CD28, CD30, CD33, CD34, CD38, CD44, CD44v6, CD44v7/8, CD45, CD45RA, CD45RO, CD56, CD62L, CD95, CD123, CD127, CD133, CD135, CD137, CD138, CD152, CD171, and wherein said equivalent is selected from the group consisting of: CCR4, CCR5, CCR6, CCR7, CXCR3, CXCR4, CXCR5, TCRαβ, TCRγδ, CTLA-4, PD1, TIM3, NKG2D, HER2/neu, ErbB, EGFR, EGFRvIII, FGFR3, FGFR4, LI-13R, II-13Rα2, II-11Rα, VEGFR2, ALK, GD2, GD3, mesothelin, survivin, PMSA, PSCA, CEA, MUC1, GPC3, GPC5, CSPG4, ROR1, FR-α, FR-β, Igk, Lewis^{Y}, glypican3, EphA2, CAIX, CSPG4, AFP, FAP, c-MET, HLA-DR, CA-125, CS1, BCMA, NKG2D ligands (MICA/MICB).

9. Product of any of the preceding claims, wherein said second entity is an aptamer, preferably an RNA aptamer.

10. Product of claim 9, wherein said aptamer is connected to said first nucleic acid by the concatenation of nucleotides resulting in a single-stranded nucleic acid molecule, preferably a single-stranded mRNA molecule, or wherein said aptamer is connected to said nucleic acid by the hybridization of complementary bases resulting in a double-stranded nucleic acid molecule, preferably a double-stranded mRNA molecule.

11. Product of any of the preceding claims further comprising nanoparticles complexed with said first and/or said second entity, and/or further comprising liposomes packaging said first and/or said second entity.

12. Product of any of claims 1-11 for the treatment a disease, preferably selected from the group consisting of: a tumor and/or oncologic disease, a hematologic disease, an infectious disease, a rheumatologic disease, a genetic/hereditary disease, an autoimmune disease, an allergic disease.

13. Medicament comprising the product of claim 12 and a pharmaceutically acceptable carrier.

14. Nucleotide-modified mRNA for an *in vivo* expression of a protein in a living being comprising
- a first ribonucleotide sequence encoding an intracellularly expressible protein, and
- a second ribonucleotide sequence encoding an aptamer configured for a specific binding to a cellular structure of said living being.
